(19) Europäisches Patentamt
European Patent Office
Office européen des brevets

(11) EP 1 157 037 B1

(12) EUROPEAN PATENT SPECIFICATION

(45) Date of publication and mention of the grant of the patent:
**30.07.2003 Bulletin 2003/31**

(21) Application number: **00904422.3**

(22) Date of filing: **19.01.2000**

(51) Int Cl.$^7$: **C07K 14/535**, A61K 47/48

(86) International application number:
**PCT/US00/01264**

(87) International publication number:
**WO 00/044785 (03.08.2000 Gazette 2000/31)**

(54) **GCSF CONJUGATES**

GCSF KONJUGATE

CONJUGUES GCSF

(84) Designated Contracting States:
**AT BE CH CY DE DK ES FI FR GB GR IE IT LI LU MC NL PT SE**
Designated Extension States:
**AL LT LV MK RO SI**

(30) Priority: **29.01.1999 US 117917 P**

(43) Date of publication of application:
**28.11.2001 Bulletin 2001/48**

(60) Divisional application:
**03007535.2**

(73) Proprietor: **F. HOFFMANN-LA ROCHE AG**
**4070 Basel (CH)**

(72) Inventor: **BAILON, Pascal, Sebastian**
**Florham Park, NJ 07932 (US)**

(74) Representative: **Brown, John David et al**
**FORRESTER & BOEHMERT**
**Pettenkoferstrasse 20-22**
**80336 München (DE)**

(56) References cited:
**EP-A- 0 401 384**
**EP-A- 0 721 958**
**WO-A-96/11953**
**US-A- 5 281 698**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 157 037 B1

## Description

### BACKGROUND OF THE INVENTION

Granulocyte colony stimulating factor (GCSF), is a pharmaceutically active protein which regulates proliferation, differentiation, and functional activation of neutrophilic granulocytes (Metcalf, Blood 67:257 (1986); Yan, et al. Blood 84(3): 795-799 (1994); Bensinger, et al. Blood 81(11): 3158-3163 (1993); Roberts, et al., Expt'1 Hematology 22: 1156-1163 (1994); Neben, et al. Blood 81(7): 1960-1967 (1993)). GCSF can mobilize stem and precursor cells from bone marrow and is used to treat patients whose granulocytes have been depleted by chemotherapy, or as a prelude to bone marrow transplants.

U.S. Patent No. 5,214,132 discloses a mutein of human GCSF which differs from native hGCSF at positions 1, 3, 4, 5, and 17, where instead of the native GCSF amino acids, the mutein has instead Ala, Thr, Tyr, Arg, and Ser respectively. (See also, Kuga, et al., Biochem. Biophys. Res. Commun. 159: 103-111 (1989)). M. Okabe, et al. (Blood 75(9): 1788-1793 (May 1, 1990)) reported a derivative of rhGCSF, in which amino acids were replaced at five positions of the N-terminal region of rhGCSF, which showed higher specific activity than intact rhGCSF in mouse and/or human bone marrow progenitor cells in two assays. U.S. Patent No. 5,218,092 discloses a mutein of human GCSF which differs from native hGCSF at positions 1, 3, 4, 5, 17, 145 and 147 where instead of the native GCSF amino acids, the mutein has instead Ala, Thr, Tyr, Arg, Ser, Asn and Ser, respectively.

The bioavailability of protein therapeutics such as GCSF is often limited by short plasma half-life and susceptibility to protease degradation, preventing maximum clinical potency. Studies of other therapeutic proteins have shown that such difficulties may be overcome by conjugating the protein to a polymer such as polyethylene glycol (PEG), typically via a linking moiety covalently bound to both the protein and the PEG.

Such PEG conjugated biomolecules have been shown to possess clinically useful properties (Inada, et al., J. Bioact. and Compatible Polymers, 5:343 (1990); Delgado, et al., Critical Reviews in Therapeutic Drug Carrier Systems, 9:249 (1992); and Katre, Advanced Drug Delivery Systems, 10:91 (1993)). Among these are better physical and thermal stability protection against susceptibility to enzymatic degradation, increased solubility, longer *in vivo* circulating half-life and decreased clearance, reduced immunogenicity and antigenicity, and reduced toxicity.

PEG-GCSF conjugates having different structures than the conjugate of this invention are disclosed in EP-A-0 335 423; EP-A-0 401 384; US-A- 5281698; EP-A-072 1958; R.W. Niven, et al., J. Controlled Release 32: 177-189 (1994); and Satake-Ishikawa, et al., Cell Structure and Function, 17:157-160 (1992)).

### SUMMARY OF THE INVENTION

Accordingly, the invention is a new class of PEG derivatives of GCSF. The conjugate of this invention has an amide linker as can be seen below.

Compared tc unmodified GCSF (i.e. GCSF without a PEG attached), the conjugate has an increased circulating half-life and plasma residence time, decreased clearance, and increased granulopoietic activity *in vivo*. In addition, compared with PEG-GCSF conjugates, the conjugate of this invention has superior granulopoietic properties. Other PEG-GCSF conjugates are disclosed in European Patent Publication No. EP 0 335 423; European Patent Publication No. EP 0 401 384; and in Niven, et al., *Ibid*. However, the conjugate of this invention has a different structure from these conjugates, and has superior properties, in particular in exhibiting long-lasting, high granulopoietic activity *in vivo* at a low dosage.

A preferred GCSF of this invention is a GCSF mutein, which has properties equivalent or superior to native GCSF and has the same uses as GCSF. The mutein has the same amino acid sequence as GCSF except at positions 1, 3, 4, 5, and 17, where instead of the native GCSF amino acids, the mutein has instead Ala, Thr, Tyr, Arg, and Ser respectively (GCSF Mutein) (See Figure 1). This mutein is disclosed in U. S. Patent No. 5,214,132, which is incorporated herein by reference.

The physiologically active PEG-GCSF conjugate of this invention has the formula

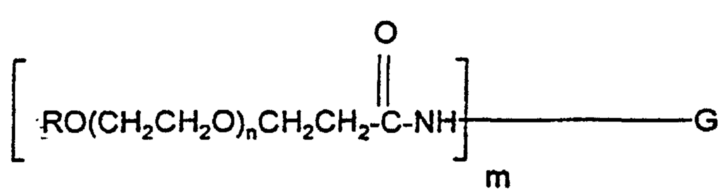

I

Also part of this invention are compositions of the claimed conjugates where m and n can be different integers for the conjugates in the composition.

The conjugate of this invention has the same uses as GCSF. In particular, the conjugate of this invention is useful to treat patients whose granulocytes have been depleted by chemotherapy or as a prelude to bone marrow transplants in the same way GCSF is used to treat these conditions. However, the conjugate of this invention has improved properties including superior stability, greater solubility, enhanced circulating half-life and plasma residence times.

## DESCRIPTION OF THE FIGURES

Figure 1: Primary Structure of GCSF Mutein
The GCSF mutein shown differs from wild type human GCSF at positions 1, 3, 4, 5, and 17, where instead of the native GCSF amino acids, the mutein has instead Ala, Thr, Tyr, Arg, and Ser respectively.

Figure 2: Pegylation Reagents

Figure 3: Separation of 20kDa PEG-modified and unmodified GCSF Mutein. A typical elution profile for PEG reaction mixture.
Column : 1-2ml Fractogel® EMD $SO_3$ 650S.
Equilibration Buffer: 10mM Ammonium Acetate, pH 4.5
Elution Buffers: 1. 0.15M NaCl in equilibration buffer 2. 0.5M NaCl in equilibration buffer

Figure 4: PEG-GCSF Mutein Activity on Day 5 after a Single Injection Female C57BL/6J mice were injected subcutaneously with 25.2µg of the pegylated GCSF Mutein conjugates; on the fifth day following administration, venous blood samples were collected from retroorbital sinus. Coulter hematological and leukocyte differential analyses were performed; the resulting neutrophil counts were standardized to vehicle control for each experiment. Data shown represent the mean ± S.E. of 4 mice per group.

Figure 5: Increase in PMN counts as a function of PEG mass (kDa) in amide and urea linked GCSF Mutein- PEG conjugates. For conjugates made with SPA reagent PMN=0.277MW+3.95. For conjugates made with urea reagent PMN=0.152 MW+2.74.

Figure 6: PEG- GCSF Mutein Activity on Day 7 after a Single Injection
Female C57BL/6J mice were injected subcutaneously with 25.2µg of the pegylated GCSF Mutein conjugates; on the seventh day following administrtion, retroorbital venous blood samples were collected. Coulter hematological and leukocyte differential analyses were performed; the resulting neutrophil counts were standarized to vehicle control for each experiment. Data shown represent the mean ± S.E. of 4 mice per group.

Figure 7: Murine PBSC Mobilization Colony Assay

Figure 8: Murine PBSC Mobilization Colony Assay

Figure 9: Murine PBSC Mobilization Colony Assay

Figure 10: Murine PBSC Mobilization Colony Assay

Figure 11: Murine PBSC Mobilization Colony Assay

## DETAILED DESCRIPTION OF THE INVENTION

The claimed invention is a physiologically active PEG-GCSF conjugate having the formula

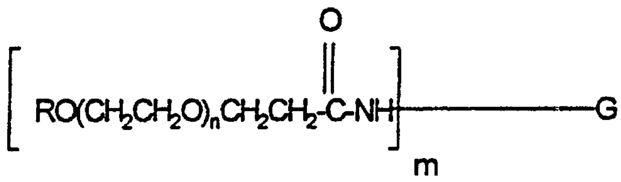

I

where G is a granulocyte colony stimulating factor less the amino groups thereof which participate in an amide bond with a polyethylene glycol moiety as shown in formula I, R is lower alkyl, n is an integer of from 420 to 550, and m is an integer from 1 to 5.

The numbers n and m are selected such that the resulting conjugate of Formula I has a physiological activity comparable to unmodified GCSF, which activity may represent the same as, more than, or a fraction of the corresponding activity of unmodified GCSF. n represents the number of ethylene oxide residues in the PEG unit. A single PEG subunit of $OCH_2CH_2$ has a molecular weight of about 44 daltons. m represents the number of PEG units attached to the GCSF molecule. A conjugate of this invention may have one, two, three, four, five or six PEG units per molecule of GCSF. Thus, the molecular weight of the conjugate (excluding the molecular weight of the GCSF) depends on the numbers n and m.

n may have a value of 420 to 550, producing a conjugate in which each PEG unit has an average molecular weight of from about 18 kilodaltons to about 25 kilodaltons per PEG unit. Preferably, n has a value of 450 to 490, producing a conjugate in which each PEG unit has an average molecular weight of about 20 kilodaltons. m may have a value of 1, 2, 3, 4, or 5. A preferred m is 1-4, and an especially preferred m is 2. The molecular weight range of the PEG portion of the conjugates of this invention is from about 18 kilodaltons (n=420, m=1) to about 125 kilodaltons (n=550, m=5). When n is from 420 to 550 and m is an integer from 1 to 4, the molecular weight range of the PEG portion of the conjugates of this invention is from about 18 kilodaltons (n=420 m=1) to about 97 kilodaltons (n=550; m=4). A molecular weight of "about" a certain number means that it is within a reasonable range of that number as determined by conventional analytical techniques.

In a preferred conjugate n is 450 to 490 and m is 1-4, in which case the molecular weight range of the PEG portion of the conjugates is from about 20 kilodaltons (n=450; m=1) to about 86 kilodaltons (n=90; m=4). In another preferred conjugate n is 420 to 550 and m is 2, in which case the molecular weight range of the PEG portion of the conjugates is from about 37 kilodaltons (n=420) to about 48 kilodaltons (n=550). In an especially preferred conjugate n is 450 to 490 and m is 2, in which case the molecular weight range of the PEG portion of the conjugates is from about 40 kilodaltons (n=450) to about 43 kilodaltons (n=490).

R may be any lower alkyl, by which is meant an alkyl group having from one to six carbon atoms such as methyl, ethyl, isopropyl, etc. Branched alkyls are included. A preferred alkyl is methyl.

By GCSF is meant the natural or recombinant protein, preferably human, as obtained from any conventional source such as tissues, protein synthesis, cell culture with natural or recombinant cells. Any protein having the activity of GCSF, such as muteins or otherwise modified proteins, is encompassed. Obtaining and isolating GCSF from natural or recombinant sources is well known (See, for example U.S. Patent Nos. 4,810,643, and 5,532,341, the contents of which are incorporated herein by reference). A preferred GCSF conjugate is a conjugate with GCSF Mutein as described in U.S. Patent No. 5,214,132.

The physiologically active conjugate of Formula I has GCSF activity, by which is meant any fraction or multiple of any known GCSF activity, as determined by various assays known in the art. In particular, the conjugate of this invention have GCSF activity as shown by the ability to increase PMN count. This is a known activity of GCSF. Such activity in a conjugate can be determined by assays well known in the art, for example the assays described below (See also: Asano, et al., Jpn. Pharmacol. Ther. (1991) 19:2767-2773; Yamasaki et al., J. Biochem. (1994) 115: 814-819; and Neben, et al., Blood (1993) 81:1960.

The conjugate of Formula I is produced by covalent linkage of a GCSF with a succinimidyl propionic acid (SPA) reagent of the formula

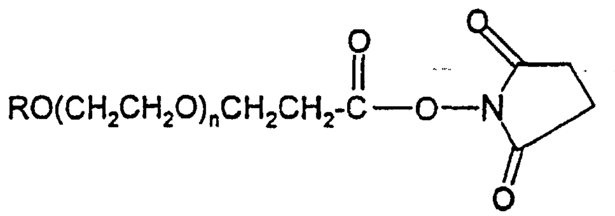

II

The reagent of formula II may be obtained by conventional methods, according to known procedures (See U.S. Patent No. 5,672,662, the contents of which are hereby incorporated by reference). n is the same as in formula I above, and is selected to produce a conjugate of the desired molecular weight. Such reagents in which n is from 450 to 490 (MW=20 kDa) are preferred. Other molecular weights may be obtained by varying n for the PEG-alcohol starting materials for the reagent of Formula II, by conventional methods. The SPA reagent of formula II in molecular weights of 5, 10, 15 and 20 kDa may be obtained from Shearwater Polymers, Inc. (Huntsville, Alabama).

The reagent of formula II may be conjugated to GCSF by conventional methods. Linkage is via an amide bond. Specifically, the reagent of Formula II primarily reacts with one or more of the primary amino groups (for example N-terminus and the lysine side chains) of GCSF to form an amide linkage between the GCSF and the polymer backbone of PEG. The NH shown in Formula I is derived from these primary amino group(s) of GCSF which react with the reagent of Formula II to form an amide bond. To a lesser degree the reagent of Formula II can also react with the hydroxy group of the Serine at position 66 of GCSF to form an ester linkage between the GCSF and the polymer backbone of PEG. The reaction conditions are conventional to a skilled person, and are provided in detail below.

Attaching the reagents to GCSF may be accomplished by conventional methods. PEGs of any selected MW of this invention may be used (n). For example, the reaction can be carried out in solution at a pH of from 5 to 10, at temperature from 4°C to room temperature, for 30 minutes to 20 hours, utilizing a molar ratio of reagent to protein of from 4:1 to 30:1. Reaction conditions may be selected to direct the reaction towards producing predominantly a desired degree of substitution. In general, low temperature, low pH (eg. pH5), and short reaction time tend to decrease the number of PEGs attached (lower m). High temperature, neutral to high pH (eg pH≥ 7), and longer reaction time to increase the number of PEGs attached (higher m). For example, in the case of the 5kDa reagent of formula II, at pH7.3 and a reagent to protein molar ratio of 30:1, a temperature of 4°C and reaction time of 30 minutes produced predominantly the mono-PEG conjugate; a temperature of 4°C and a reaction time of 4 hours produced predominantly the di-PEG conjugate; and a temperature of room temperature and a reaction time of 4 hours produced predominantly the tri-PEG conjugate. The reaction is terminated by acidifying the reaction mixture and freezing at -20°C. In general a pH of from 7 to 7.5, and a reagent to protein molar ratio of from 4:1 to 6:1, are preferred.

Purification methods such as cation exchange chromatography may be used to separate conjugates by charge difference, which effectively separates ccnjugates into their various molecular weights. For example, the cation exchange column can be loaded and then washed with ~20mM sodium acetate, pH~4, and then eluted with a linear (0M to 0.5M) NaCl gradient buffered at a pH from 3 to 5.5, preferably at pH~4.5. The content of the fractions obtained by cation exchange chromatography may be identified by molecular weight using conventional methods, for example, mass spectroscopy, SDS-PAGE, or other known methods for separating molecular entities by molecular weight. A fraction then is accordingly identified which contains the conjugate of Formula I having the desired number (m) of PEGs attached, purified free from unmodified GCSF and from conjugates having other numbers of PEGs attached.

Also part of this invention is a composition of conjugates where conjugates having different values of m are included in specific ratios. A preferred composition of this invention is a mixture of conjugates where m=1, 2, 3 and 4. The percentage of conjugates where m=1 is 18-25%, the percentage of conjugates where m=2 is 50-66%, the percentage of conjugates where m=3 is 12-16%, and the percentage of conjugates where m=4 is up to 5%. Such a composition is produced by reacting pegylation reagent with GCSF in a molar ratio of from 4 to 6:1 (excess reagent). The reaction is allowed to proceed at 4°C to 8°C for 20 hours at pH near 7.5. At the end of the reaction, acetic acid is added. The conjugate is then purified from residual unmodified protein, excess pegylation reagent and other impurities and buffer components present during the reaction. Along with pegylated protein, N-hydroxysuccinimide and polyethylene glycol-carboxylic acid are produced as reaction byproducts.

The following Examples are provided to illustrate the invention described herein, and do not limit it in any way. GCSF Mutein is used in these examples. Other species of GCSF may also be conjugated to PEG by the methods exemplified.

**Example 1**

**Pegylation Reagents:**

1. GABA Amide Linker (P-6GA-1, P-12Ga-1)
The GABA Amide linker reagents, contain 2 PEG strands of either 6 or 12kDa. See Figure 2-A for the structures.

2. Amide Linker (P-5 am-1, P-10am-1)
Five and 10kDa amide linkers were produced. See Figure 2-B for the structure.

3. Amide Linker
This reagent was a commercial succinimidyl propionic acid (SPA), prepared with 5, 10, 15 and 20kDa PEG molecules, and their general structure is illustrated in Figure 2-C.

4. Urea Linker
This reagent was prepared with 5, 10 and 25kDa PEG molecules and the typical structure is illustrated in Figure 2-D.

5. Urethane Linker
Ten and 20kDa urethane linkers were produced and the structure is shown in Figure 2-E.

6. Urethane Linker
As the structure of this commercially prepared 36kDa PEG reagent, illustrated in Figure 2-G, indicated one end of the PEG reagent is capped with a t-butyl group. This reagent was the highest M.W. PEG used in this example.

7. Thio-urethane Linker
This pegylation reagent structure can been seen in Figure 2-F. The M.W. of the PEG used in this reagent was 20kDa.

The following reagents were provided by Kyowa Hakko Kogyo Co., Ltd. Tokyo, Japan): 1) G-CSF mutein denoted GCSF Mutein, GCSF Mutein conjugated to a branched methoxy polyethylene glycol (m-PEG) reagent comprising of 2 m-PEG chains of either 6 or 12 kDa (PEG-GABA-NHS, see Fig. 2A) GCSF Mutein conjugated to 5 and 10kDa linear, ester/amide m-PEG reagent (see Fig. 2B), m-PEG-Succinimidyl propionic acid-NHS (PEG-SPA) reagents having molecular weights of 5, 10 15 and 20kDa were purchased from Shearwater Polymers, (Huntsville, Alabama, see Fig. 2C). The following protein pegylation reagents were prepared at Hoffmann-La Roche, Inc: 1) m-PEG-urea linker (5, 10 and 25kDa, see Fig. 2D), 2)m-PEG-urethane linker (10 and 20kDa, see Fig. 2E) m-PEG-thiourethane linker (10 and 20kDa see Fig. 2F) and The t-butyl- m-PEG-urethane linker reagent with an average M.W. of 36kDa was obtained from DDI Pharmaceuticals, Inc. (Mountainview, CA, see Fig. 2G).

**Pegylation Reactions**

The factors which affect the pegylation reactions are 1) pH, 2) temperature, 3) time of reaction, 4) protein to PEG reagent molar ratio, and 5) protein concentration. By controlling one or more of these factors, one can direct the reaction towards producing predominantly mono-, di-, tri-, etc. PEG conjugates. For example, the reaction conditions for Shearwater Polymer's SPA-PEG 5000 (N-hydroxy succinimide) reagent were 1) pH 7.3, 2) temperature 4°C, for mono- and di-PEG, and room temperature for tri-PEG, 3) time of reaction for mono-PEG, 30 minutes; for di- and tri-PEG, 4 hours and 4) protein to reagent molar ratio of 1:30. For all reagents, the optimal reaction conditions to produce the desired PEG species were determined individually. They are shown in Table 1. The reaction is terminated by acidifying the reaction mixture and freezing at -20°C.

**Separating Modified and Free GCSF Mutein from the Reaction Mixture (Sulfopropyl (SP) Cation Exchange)**

The reaction mixture, containing approximately 5mg protein, was diluted 10 to 20-fold with water and the pH adjusted to 4.5 with glacial acetic acid. The diluted sample was then applied to a previously packed 1-2ml Fractogel EMD $SO_3$ -- 650S (EM Separations, Gibbstown, New Jersey) column, which was equilibrated with 10mM ammonium acetate, pH 4.5 The unadsorbed reagent and reaction byproducts were removed in the flowthrough. The modified GCSF Mutein was eluted with a step gradient using 0.15M NaCl in the equilibration buffer. The unmodified GCSF Mutein remaining on the column was step-eluted with 0.5M NaCl in the equilibration buffer. The separated GCSF

Mutein-PEG conjugate mixture was sterile filtered with a 0.2μm filter and stored frozen at -20°C.

**Characterization of GCSF Mutein PEG congugates**

Protein Determination

Protein concentrations of the purified GCSF Mutein PEG conjugates were determined using an $A_{280}$ value of 0.86, for a 1mg/ml solution.

SDS-PAGE Analysis

This analysis was performed using 12 and 15% polyacrylamide gels or 8-16% polyacrylamide gradient gels, under reducing conditions,according to Laemmli, Nature 227:680-685, 1970.

Percent Composition Determination

The percent composition of each species (mono-, di-, tri-, etc.) in the various GCSF Mutein-PEG conjugate reaction mixtures was determined from the densitometric measurements of Coomassie blue-stained SDS-PAGE gels (see Table 2).

Determination of PEG mass in GCSF Mutein PEG conjugates

The total mass of PEG substituted in various preparations was determined from the average PEG molecular weight, identification of individual PEG conjugates (mono, di etc.), based upon elecrophoretic mobility, the number of PEG molecules attached, and the percent composition based on densitometric measurements of Coomassie blue stained SDS-PAGE. The total PEG mass of a particular preparation is the sum of its individual PEG masses. The individual PEG mass is calculated from the following equation:

$$\text{PEG mass} = \text{PEG M.W.} \times \#\ \text{PEG molecules} \times \%\ \text{Composition}$$

where

PEG M.W. - 5, 10, 20 kDa, etc.
# PEG Molecules = 1, 2, 3 for mono, di, tri, respectively.

Mass spectrometry (MALDI-TOF) has also been used in the total PEG mass determination. In this instance, the mass spectrum allowed the identification and the determination of the molecular weight of individual PEG conjugates. The PEG M.W. attached to each PEG conjugate is the total M.W. of individual PEG conjugates minus the M.W. of GCSF Mutein (18.9kDa). These values multiplied by % composition, yield individual PEG masses; their sum is the total PEG mass.

Both methods have been utilized for determining the PEG masses of various preparation. The results are summarized in Table 2.

Determination of Endotoxin Levels

Endotoxin levels were determined using the LAL method, according to the manufacturer's instructions (Associates of Cape Cod, Inc., Woods Hople, Massachusetts).

Bioactivities

The *in vitro* bioassay on M-NFS-60 cells and the *in vivo* assay in female C57BL/6J mice were performed as previously described. (See Asano, et al., Jpn. Pharmacol. Ther. (1991) 19:2767-2773.)

### Results and Discussion

### Pegylation Reaction

Generally, results indicate that less reactive reagents, such as the urea linker, require higher pH, temperature, and protein:reagent molar ratio, as well as longer reaction time, to obtain the desired amount of conjugation (see Tables 1 and 2).

### Separation of Modified and Free GCSF Mutein from the Reaction Mixture

A typical elution profile is shown in Figure 4. In addition to cation exchange chromatography, additional steps such as gel permeation chromatography may be required to remove trace contaminants and endotoxin, and to perform buffer exchange of the final product for storage. The strong cation exchange separation method has been scaled-up to a 30mg scale for the 20kDa SPA (amide) and 20kDa urethane conjugates. Nearly quantitative recoveries are obtained with this procedure.

### % Composition and PEG Mass

The percent composition and PEG mass results are summarized in Table 2. In our experience, in the case of high M.W. PEG conjugates (e.g. 20kDa SPA diPEG and 12 kDa GABA), identifying PEG species based upon electrophorectic mobility to determine the % composition of a reaction mixture is not very reliable. In order to determine the PEG mass, and identification of high M.W. and highly substituted PEG conjugates, a combination of SDS-PAGE, SP-HPLC and MALDI-TOF MS analyses are needed. However, monopegylated and PEG conjugates derived from low M.W. PEG reagents (e.g., 5kDa) could be identified fairly accurately from their respective SDS-PAGE profiles.

### Endotoxin Levels

Using the LAL method, < 1 EU/mg of endotoxin was detected in all PEG conjugates except the one derived from urethane reagent. In this PEG conjugate, endotoxin was detected only after dilution. It has been confirmed that this is not due to contamination during dilution and therefore some unknown material in this sample may have caused an inhibition in the LAL assay, at higher protein concentration. Upon dilution of the sample, and subsequently diluting the inhibitory material, a positive endotoxin result was observed.

### Bioactivity

*In vitro* and *in vivo* bioactivities of all GCSF Mutein PEG conjugates are listed in Table 2. Generally, an inverse relationship between the *in vitro* activity and the degree of subsitution, as well as the M.W. of the PEG, are observed. In contrast, an enhancement in *in vivo* activity is observed with increasing M.W. of the substituted PEG. This is also observed by others (Satako-Ishikawa, et al., *Cell Struct Funct*. 17(3):157-60, 1992.). It is postulated that the chemical attachment of PEG molecules to the polypeptide backbone of GCSF Mutein produces some form of conformational changes which adversely affect receptor/ligand interactions thus lowering binding affinity. In addition, the relatively short incubation time of the *in vitro* assay is probably insufficient to reach peak activity. On the other hand, the *in vivo* assay in mice is much longer (days) and is terminated several days after the injection of the drug. This longer incubation time, combined with the increased circulating half-life of the PEG-GCSF Mutein, compensate for any loss in binding affinity due to pegylation. The end-result is attainment of maximum *in vivo* bioactivity. Another hypothesis is that PEG-GCSF Mutein is acting as a prodrug when injected into mice. In this situation, the PEG moiety of PEG-GCSF Mutein is somehow continually being cleaved off, resulting in a sustained release of minute amounts of free GCSF Mutein, which accounts for the maintenance and enhancement of *in vivo* activity. However, the prodrug hypothesis does not explain the observed base line *in vivo* activity, 7 days after the initial dosing. The prodrug mechanism is unlikely because the amide bond between the protein and PEG is stable and not easily cleaved.

Among the 15 GCSF Mutein PEG conjugates studied, the *in vivo* activities of P-12GA-1, 20kDa SPA, 20kDa urethane and 36kDa urethane, were significantly higher than the rest of the preparations (See Figure 4 and Table 2).

Overall, a direct relationship between the M.W. of the PEG molecule and an increased *in vivo* activity is observed. This is illustrated in Figure 5, where the increase in PMN counts are expresed as a function of the total PEG mass in amide (SPA) and urea linked PEG conjugates.

**Selection and Characteristics of GCSF Mutein PEG Conjugates**

After careful evaluation of the conjugation chemistry, biological properties and drug development issues among the 15 PEG conjugates, the three chosen for further evaluation are: 1) P-12GA-1,2) 20kDa SPA and 3) 20kDa urethane. The 20kDa SPA-derived mono, di and triPEG conjugates present in the SP-purified reaction mixture, were evaluated in a Head-to-head compassion which showed that all three maintained high granulopoietic activity in female C57BL/6J mice for 5 days with a single dose of 25.2µg (Table 3 and Figure 4). In contrast, daily doses of the unmodified GCSF Mutein were needed to maintain similar activities (data not shown). In all but two cases (20kDa SPA and P-12GA-1), *in vivo* activity returned to normal levels on day 7 after the initial dosing of the mice (Figure 6). Both the 20kDa SPA and P-12GA-1 conjugates exhibited increased activity at the lower dosage of 8.4µg and returned to normal levels on day 7 (see Table 3). The percent composition data (Table 3) indicates that both the 20kDa SPA and P-12GA-1 preparations contain approximately 50% dimer and the remaining 50% is distributed between monomer and trimer. The 20kDa urethane reagent produces predominantly mono-PEG under the experimental conditions used (see Table 3). The *in vitro* activity of all PEG conjugates evaluated, including the predominantly monomeric urethane derivative, follows the general pattern of an inverse relationship between degree of substitution, as well as the M.W. of PEG. The *in vivo* biological activity of the PEG conjugates evaluated showed a direct relationship to the M.W. of the PEG over the molecular weight range evaluated (Figure 5).

**Conclusion**

Among the 15 GCSF Mutein PEG conjugates examined, the P-12GA-1, 20kDa SPA and the 20kDa urethane linker preparations exhibited good *in vivo* activity profiles. The 20kDa PEG-GCSF Mutein exhibited the best overall properties, including economics of production.

Table 1. Reaction Conditions Used For the Preparation of Various PEG Conjugates

| MW | Chemistry | Reaction Conditions | | | |
|---|---|---|---|---|---|
| | | PH | Temperature | Time | Ratio |
| 5k | UREA | 10 | RT | 1hr | 1:100 |
| 5k | AMIDE | 7.3 | RT | 4hrs | 1:30 |
| | | | | | |
| | | | | | |
| 10k | UREA | 10 | RT | 1hr | 1:100 |
| 10k | AMIDE | 7.3 | RT | 4hrs | 1:30 |
| 10k | URETHANE | 10 | 4 C | 1hr | 1:30 |
| | | | | | |
| | | | | | |
| 15k | AMIDE | 7.3 | RT | 4hrs | 1:30 |
| | | | | | |
| | | | | | |
| 20k | AMIDE | 7.3 | RT | 4hrs | 1:30 |
| 20k | THIO-URETH. | 8 | RT | 17hrs | 1:30 |
| 20k | URETHANE | 10 | 4 C | 1hr | 1:30 |
| | | | | | |
| 25k | UREA | 10 | RT | 1hr | 1:100 |
| | | | | | |
| 36k | URETHANE | 8 | 4 C | 6hrs | 1:3 |

Table 2. Composition, PEG Mass and Bioactivity Data of Various PEG-GCSF Mutein Conjugates

| | | | Activities | | |
|---|---|---|---|---|---|
| Linker Type | * % Composition mono di tri oligo | * Mass of PEG Added | M-NFS-60 | WBC | PMN (% of ctrl) |
| *Amide* | | | | | |
| (b) 5K | 17.3 22.3 51.3 9.1 | 12600** | 9% | 22.48 | 536+40 |
| (a) 5K | 0 0 0 100 | 20000 | 5% | 18.65 | 539+23 |
| (b) 10K | 9.8 63 27.2 0 | 21700** | 11% | 20.48 | 632+82 |
| (a) 10K | 10 11 53 26 | 29500 | 4% | 20.13 | 701+92 |
| (b) 15 K | 13.7 61.2 25.1 0 | 31710 | 6% | 26.68 | 751+115 |
| (c) 20 K | 27.6 49.5 22.9 0 | 38100** | 5% | 29.23 | 977+120 |
| *Urea* | | | | | |
| (a) 5K | 28 19 23 23 | 11350 | 40% | 12.78 | 254+27 |
| (a) 10K | 29 19 23 23 | 22800 | 42% | 14.4 | 364+50 |
| (b) 25k | 24.7 15.4 41.6 18.7 | 63775 | 6% | 27.78 | 716+87 |
| *Urethane* | | | | | |
| (b) 10K | 15.8 12.6 36.5 35 | 29050 | 10% | 19.9 | 412+88 |
| (c) 20K | 81.8 4 14.2 0 | 26800** | 7% | 25.83 | 656+52 |
| (a) 36K | 50 50 0 0 | 54000 | 15% | 25.05 | 888+132 |
| *Thio-Urethane* | | | | | |
| (b) 20K | 70.8 12 17.3 0 | 28440 | 20% | 21.85 | 494+71 |
| *GABA* | | | | | |
| (b) 5K | 43.4 54.3 2.3 0 | 18100** | 11% | 29 | 598+117 |
| (c) 12K | 36 47 17 0 | 46500** | 3% | 30.03 | 886+120 |

% Composition and Mass of PEG added are calculated based on densitometric measurements of Coomassie-blue stained SDS-PAGE (*), OR by MALDI TOF MAS ANALYSIS (**).

(a), (b), (c): Each representing a separate bioassay; Day 5 PMN data reported

Table 3. Composition, PEG Mass, Pegylated Sites and Bioactivity Data of the Three Lead Molecules

| PEG Molecule | *% Composition | | | | **PEG Mass (kDa) | Activities | | | | | | | | |
|---|---|---|---|---|---|---|---|---|---|---|---|---|---|---|
| | mono | di | tri | oligo | | M-NFS | WBC | | | | PMN (% of control) | | | |
| | | | | | | 60 | Day 5 | | Day 7 | | Day 5 | | Day 7 | |
| Vehicle (control) | | | | | | | 8.78 | | 8.97 | | 100 | | 100 | |
| ND-28: Single Injection of 25.2ug | | | | | | | 8.05 | | 6.1 | | 86 | | 76 | |
| ND-28. Daily Injection of 25.2ug | | | | | | | 26.5 | | 24.58 | | 1182 | | 906 | |
| | | | | | | | Dosage | | | | | | | |
| | | | | | | | 8.4ug | 25.2ug | 8.4ug | 25.2ug | 8.4ug | 25.2ug | 8.4ug | 25.2ug |
| 12K GABA | 36.0 | 47.0 | 17.0 | 0.0 | 46.5 | 3% | 22.75 | 30.03 | 10.7 | 24.1 | 701 | 1064 | 140 | 556 |
| 20K SPA | 27.6 | 49.5 | 22.9 | 0.0 | 38.1 | 5% | 13.58 | 21.63 | 7.5 | 15.45 | 519 | 978 | 103 | 447 |
| 20K Urethane | 81.8 | 4.0 | 14.2 | 0.0 | 26.8 | 7% | 8.35 | 17.43 | 6.78 | 7.93 | 222 | 729 | 74 | 119 |

Female C57BL/6J mice were administered 8.4 or 25.2ug of either ND-28 daily or a single dose of PEG conjugate.

On day 5 and day 7 following initiation of dosing, venous blood samples were taken and differential leukocyte analysis was performed.

*Based on Densitometric measurements of Coomassie-stained SDS-PAGE

**Determined by MALDI TOF MS

**Example 2: Preparation of 20 kDa PEG Conjugated to rhG-CSF Mutein**

Modification of G-CSF mutein with 20 kDa methoxy-PEG succinimidyl propionic acid (SPA) was performed as follows. PEG reagent was dissolved in distilled water at a concentration of ~200 mg/ml and added to the G-CSF mutein solution (~5mg/ml) in a molar ratio of from 4:1 to 6:1 (excess reagent). The reaction was allowed to proceed at 4°C to 8°C for 20 hours at pH ~7.5. At the end of the reaction, glacial acetic acid was added to stop the reaction. Pegylated GCSF Mutein (also referred to as PEGG) was then purified from residual unmodified mutein, excess PEG reagent, and other impurities and buffer components present during the modification. Along with pegylated protein, N-hydroxysuccinimide and polyethylene glycol-carboxylic acid are produced as reaction byproducts.

PEGG was purified using cation exchange chromatography followed by ultrafiltration. The cation exchange column was loaded and washed with 20 mM sodium acetate, pH 4.0. Elution with a linear sodium chloride gradient separated PEGG from all other components in the reaction mixture. Subsequently, ultrafiltration/diafiltration was used to concentrate the PEGG to ~4.0 mg/mL and to change the buffer to 20 mM sodium acetate, 50 mM sodium chloride, pH 6.0.

Five pegylations and purification runs carried out under the conditions listed above were analyzed using cation exchange chromatography, and this has demonstrated the reproducibility of the G-CSF mutein pegylation reaction. The pegylation reaction was demonstrated to be reproducible in runs up to 2.5 g (final PEGG yield) under the following optimum conditions: 20kDa-SPA-PEG:mutein ratio of 4 to 6:1; pH ~7.5, 4°C, 20 hours. The average percent composition of the mixture of PEGG's was determined to be 21.7% mono-PEGG (%RSD = 16.6), 60.3% di-PEGG (%RSD =6.6), 15.1% tri-PEGG (%RSD=4.0), and 2.9% tetra-PEGG (%RSD=26.1), as shown in Table 4.

Table 4:

| Cation Exchange Analysis of Relative Percent composition of Mono, Di, Tri, and Tetra-PEGG in Five PEGG Syntheses and Purification Runs | | | | |
|---|---|---|---|---|
| Run Number | Mono-PEGG (%RSD, Five determinations) | Di-PEGG (%RSD, Five determinations) | Tri-PEGG (%RSD, Five determinations) | Tetra-PEGG (%RSD, Five determinations) |
| 1 | 21.9% (8.0) | 60.3% (2.2) | 15.1% (2.2) | 2.7% (4.7) |
| 2 | 27.5% (2.3) | 54.4% (1.0) | 15.7% (0.8) | 2.4% (1.2) |
| 3 | 18.2% (7.1) | 65.5% (0.6) | 14.3% (6.6) | 2.0% (9.3) |
| 4 | 21.7% (2.7) | 60.1% (1.0) | 14.8% (0.5) | 3.5% (Q.9) |
| 5 | 19.2% (1.8) | 61.3% (0.9) | 15.7% (3.7) | 3.8% (4.5) |
| Averaged Composition | 21.7% (16.6) | 60.3% (6.6) | 15.1%(4.0) | 2.9%(26.1) |

**Example 3**

**Peripheral Blood Stem Cell Mobilization**

Techniques have been developed to mobilize both primitive stem cells and committed precursors from bone marrow, and to expand circulating progenitor cells in peripheral blood. These stimulated cells may be capable of mediating early and sustained engraftment following lethal irradiation and bone marrow or stem cell transplant. Neben, S. Marcus, K and Mauch, P: Mobilization of hematopoietic stem and progenitor cell subpopulations from the marrow to the blood of mice following cyclophosphamide and/or granulocyte colony-stimulating factor. Blood 81: 1960 (1993). The recruitment of peripheral blood stem cells (PBSC) can help shorten hematopoietic recovery in patients with chemotherpay-induced bone marrow hypoplasia or those undergoing other myeloablative treatments. Roberts, AW and Metcalf, D: Granulocyte colony-stimulating factor induces selective elevations of progenitor cells in the peripheral blood of mice. **Experimental Hematology** 22: 1156 (1994). Both growth factors and chemotherapeutic drugs have been used to stimulate mobilization. Bodine, D: Mobilization of peripheral blood "stem" cells: where there is smoke, is there fire? **Experimental Hematology** 23: 293 (1995). Following stimulation of PBSC, the mobilized cells are harvested by leukapheresis and cryopreserved until such time as they are needed. Current clinical protocols call for repeated collection of PBSC concentrates by leukapheresis following standard high-dose chemotherapy (CHT) and repeated daily dosing or continuous infusion with growth factors, sometimes lasting two weeks or more. Brugger, W, Bross, K, Frisch, J, Dern, P, Weber, B, Mertelsmann, R and Kanz, L: Mobilization of peripheral blood progenitor cells by sequential

administration of Interleukin-3 and granulocyte-macrophage colony-stimulating factor following polychemotherapy with etoposide, ifosfamide, and cisplatin. **Blood** 79: 1193 (1992). The studies described below were performed with two mouse models of PBSC mobilization, the first in normal mice, and the second in a chemotherapy model. The experiments demonstrate the increased efficacy of pegylated G-CSF Mutein in accordance with this invention (PEGG), as compared to NEUPOGEN (G-CSF), to effect mobilization of stem cells. The superiority of the pegylated mutein in a significantly reduced and more efficient dosing regimen is clearly established as well.

These studies evaluated the expansion capacity of mobilized immature murine PBSC stimulated *in vitro* with multiple growth factors in a seven day agar colony assay. In addition to colony-forming ability, a complete hematological profile plus an evaluation of absolute neutrophil counts (ANC) was performed on the blood of all mice. Serum G-CSF levels were determined as well. Following assay optimization, several time course experiments were performed, and high and low doses of G-CSF were examined. The experimental models included G-CSF- and cyclophosphamide (Cytoxan)-induced mobilization, as well as a combination treatment using both CHT and the cytokine.

*Materials and Methods:* 6- to 10-week-old female C57BL/6J mice, purchased from The Jackson Laboratory, were used in all experiments. The mice were injected IP on day -1 with either 200 mg/kg Cytoxan, or phosphate buffered saline (PBS) vehicle. On day 0, the animals were injected SC with 0.1 ml of either NEUPOGEN (GCSF), PEGG (20 kD SPA-linked pegylated mutein, Lot #P20W3), or PBS vehicle containing 1% normal mouse serum. Mice receiving Neupogen were given daily injections of the same dose, while all other mice received vehicle. On the day of sacrifice, peripheral blood was collected from the retroorbital sinus of anesthetized mice into EDTA-containing tubes. For each group, a small volume of pooled whole blood was added to triplicate $35mm^2$ tissue culture dishes containing 1000 U/ml recombinant mouse (rm) Interleukin-3, 100 ng/ml rm stem cell factor, and 1000 U/ml rm Interleukin-6, in a toal of 1 ml RPMI 1640 medium supplemented with 15% fetal bovine serum and 0.35% and 0.35% Difco agar. The solidified agar cultures were incubated for one week at 37°C in a humidified 5% $CO_2$ in air atmosphere. Colonies were enumerated using a stereo dissecting microscope under dark field illumination.

*Results:* In the first study shown, normal mice received daily injections of 25 μg/mouse NEUPOGEN on days 0 - 5, or a single injection of 25 μg/mouse PEGG on day 0. Mice were sacrificed on days 3 - 7. As seen in Figure 7, mobilization as demonstrated by colony formation was significantly increased in NEUPOGEN-injected mice on days 3 and 4, but gradually began to return to baseline levels by day 5 (despite NEUPOGEN injections through day 5). Mice injected with PEGG, on the other hand, demonstrated more highly evaluated numbers of colonies, which remained at plateau levels through day 7.

The paradigm for the chemotherapy model was similar. Mice in the CHT groups received an injection of Cytoxan on day -1. Some mice then received only vehicle on subsequent days, while others received a combination treatment of either daily injections of NEUPOGEN on days 0 - 5, or a single injection of PEGG on day 0. Figure 8 shows a peak in Cytoxan treated mice on day 4, with a gradual return to baseline levels on subsequent days. Both the NEUPOGEN and PEGG groups peaked on day 5, demonstrating highly elevated colony numbers. However, the Cytoxan + PEGG values remained very significantly elevated over those in the Cytoxan + NEUPOGEN group through days 6 and 7. Figure 9 demonstrates the synergistic effect of combination therapy over that of Cytoxan or G-CSF alone.

A second study is shown in Figures 10 and 11. Normal mice receiving daily injections of a lower, 3 μg/mouse dose of NEUPOGEN for 10 consecutive days demonstrated a relatively low level of "multiphasic" mobilization throughout the time course examined. Animals injected with a single 3 μg/mouse dose of PEGG displayed approximately five times that number of mobilized progenitors in the peripheral circulation by day 4, although the effect was single burst which was essentially over within 6 days.

In Cytoxan-injected mice, a single dose of 3 μg/mouse PEGG induced roughly an equivalent amount of PBSC mobilization as 30 μg/mouse of NEUPOGEN injected in 10 daily doses of 3 μg/day (Fig. 11). Both groups peaked in mobilization of progenitors on day 5, and the magnitude of the peaks was identical. The only difference appeared to be a slightly longer lingering effect of NEUPOGEN. The numbers of colonies in the CHT model were 4 - 10 times higher than those in the normal mouse model.

These experiments demonstrate two distinct potential advantages of pegylated mutein over NEUPOGEN. First, the greater efficacy of PEGG compared to NEUPOGEN in ability to induce mobilization of PBSC is evident, in both normal and chemotherapy-treated mice. Second, the pegylated mutein has been shown to be more effective than NEUPOGEN in mice, using a reduced and more efficient dosing regimen than that currently utilized with the clinical product.

## Claims

**1.** A physiologically active conjugate having the formula

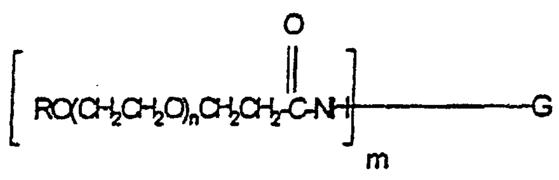

I

wherein G is a granulocyte colony stimulating factor less the amino group or groups thereof which form an amide linkage with a polyethylene glycol moiety in the conjugate; R is lower alkyl; n is an integer from 420 to *550;* and m is an integer from 1 to 5.

**2.** The conjugate of claim 1 wherein R is methyl.

**3.** The conjugate of claim 2 wherein n is from 450 to 490.

**4.** The conjugate of claim 2 wherein m is an integer from 1 to 4.

**5.** The conjugate of claim 4 wherein m is 2.

**6.** The conjugate of claim 1 wherein the granulocyte colony stimulating factor is GCSF Mutein having the sequence shown in Figure 1.

**7.** The conjugate of claim 1 wherein n is from 450 to 490.

**8.** The conjugate of claim 1 wherein m is an integer from 1 to 4.

**9.** The conjugate of claim 8 wherein m is 2.

**10.** The conjugate of claim 1 wherein the granulocyte colony stimulating factor is GCSF Mutein having the sequence shown in Figure 1.

**11.** The conjugate of claim 1, which has a longer circulating half-life and greater in vivo granulopoietic activity than the corresponding unconjugated granulocyte colony stimulating factor.

**12.** The conjugate of claim 11, wherein the granulocyte colony stimulating factor is GCSF Mutein having the sequence shown in Figure 1.

**13.** A conjugate according to claim 1
wherein R is methyl; n is an integer from 450 to 490; m is 2; and G is GCSF Mutein having the sequence shown in Figure 1 less the amino groups thereof which form an amide linkage with a polyethylene glycol moiety in the conjugate.

**14.** A composition comprising physiologically active conjugates having the formula

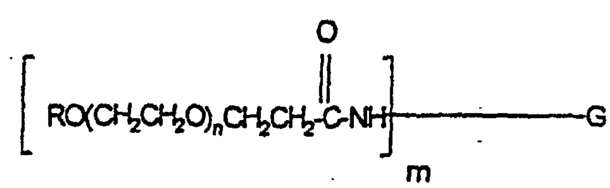

I

wherein G in each of the conjugates is a granulocyte colony stimulating factor less the amino group or groups thereof which form an amide linkage with a polyethylene glycol moiety in the conjugates; R in each of the conjugates

is independently lower alkyl; n in each of the conjugates is independently an integer from 420 to 550; in each of the conjugates m is independently an integer from 1 to 4; the percentage of conjugates where m is 1 is from eighteen to twenty-five percent; the percentage of conjugates where m is 2 is from fifty to sixty-six percent; the percentage of conjugates where m is 3 is from twelve to sixteen percent; and the percentage of conjugates where m is 4 is up to five percent.

**15.** The composition of claim 14, wherein R is methyl in each of the conjugates.

**16.** The composition of claim 14, wherein n and R are the same in each of the conjugates.

**17.** The composition of claim 14 wherein n is from 450 to 490.

**18.** The composition of claim 14 where in each of the conjugates the granulocyte colony stimulating factor is GCSF Mutein having the sequence shown in Figure 1.

**19.** A composition according to claim 14
wherein R is methyl; n in each of the conjugates is the same and is an integer from 450 to 490; G is GCSF Mutein having the sequence shown in Figure 1 less the amino groups thereof which form an amide linkage with a polyethylene glycol moiety in the conjugate; in each of the conjugates m is independently an integer from 1 to 4; the percentage of conjugates where m is 1 is from eighteen to twenty-five percent; the percentage of conjugates where m is 2 is from fifty to sixty-six percent; the percentage of conjugates where m is 3 is from twelve to sixteen percent; and the percentage of conjugates where m is 4 is up to five percent.

**20.** A method for producing a PEG-GCSF conjugate having a longer circulating half-life and greater *in vivo* granulopoietic activity than the corresponding unconjugated GCSF, which method consists of covalently linking a reagent of the formula

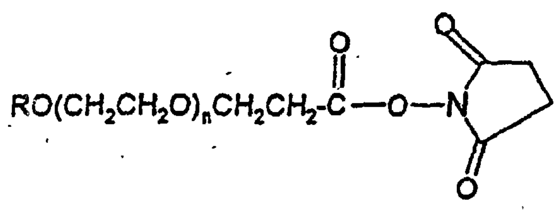

II

to the GCSF to produce said conjugate.

**21.** A method according to claim 20, wherein the GCSF is GCSF Mutein having the sequence shown in Figure 1.

**Patentansprüche**

**1.** Physiologisch aktives Konjugat der Formel

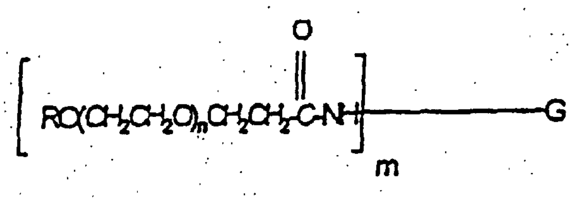

I

wobei G ein Granulocyten-Kolonie-stimulierender Faktor ist, abzüglich der Aminogruppe oder -gruppen desselben,

die eine Amidbindung mit einer Polyethylenglycol-Einheit im Konjugat bilden; R ein kurzkettiges Alkyl ist; n eine ganze Zahl von 420 bis 550 ist; und m eine ganze Zahl von 1 bis 5 ist.

**2.** Konjugat nach Anspruch 1, wobei R Methyl ist.

**3.** Konjugat nach Anspruch 2, wobei n von 450 bis 490 ist.

**4.** Konjugat nach Anspruch 2, wobei m eine ganze Zahl von 1 bis 4 ist.

**5.** Konjugat nach Anspruch 4, wobei m 2 ist.

**6.** Konjugat nach Anspruch 1, wobei der Granulocyten-Kolonie-stimulierende Faktor GCSF-Mutein ist, das die in Figur 1 gezeigte Sequenz hat.

**7.** Konjugat nach Anspruch 1, wobei n von 450 bis 490 ist.

**8.** Konjugat nach Anspruch 1, wobei m eine ganze Zahl von 1 bis 4 ist.

**9.** Konjugat nach Anspruch 8, wobei m 2 ist.

**10.** Konjugat nach Anspruch 1, wobei der Granulocyten-Kolonie-stimulierende Faktor GCSF-Mutein ist, das die in Figur 1 gezeigte Sequenz hat.

**11.** Konjugat nach Anspruch 1, das eine längere Zirkulations-Halbwertszeit und eine größere *in vivo*-granulopoietische Aktivität hat als der entsprechende, nicht-konjugierte Granulocyten-Kolonie-stimulierende Faktor.

**12.** Konjugat nach Anspruch 11, wobei der Granulocyten-Kolonie-stimulierende Faktor GCSF-Mutein ist, das die in Figur 1 gezeigte Sequenz hat.

**13.** Konjugat nach Anspruch 1, wobei R Methyl ist; n eine ganze Zahl von 450 bis 490 ist; m 2 ist; und G GCSF-Mutein ist, das die in Figur 1 gezeigte Sequenz hat, abzüglich der Aminogruppen desselben, die eine Amidbindung mit einer Polyethylenglycol-Einheit im Konjugat bilden.

**14.** Zusammensetzung, umfassend physiologisch aktive Konjugate der Formel

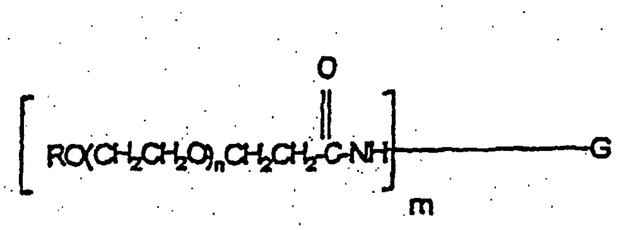

I

wobei G in jedem der Konjugate ein Granulocyten-Kolonie-stimulierender Faktor ist, abzüglich der Aminogruppe oder -gruppen desselben, die eine Amidbindung mit einer Polyethylenglycol-Einheit in den Konjugaten bilden; R in jedem der Konjugate unabhängig voneinander ein kurzkettiges Alkyl ist; n in jedem der Konjugate unabhängig voneinander eine ganze Zahl von 420 bis 550 ist; in jedem der Konjugate m unabhängig voneinander eine ganze Zahl von 1 bis 4 ist; der prozentuale Anteil an Konjugaten, bei denen m 1 ist, von 18-25% beträgt, der prozentuale Anteil an Konjugaten, bei denen m 2 ist, von 50 bis 66% beträgt; der prozentuale Anteil an Konjugaten, bei denen m 3 ist, von 12 bis 16% beträgt; und der prozentuale Anteil an Konjugaten, bei denen m 4 ist, bis zu 5% beträgt.

**15.** Zusammensetzung nach Anspruch 14, wobei R in jedem der Konjugate Methyl ist.

**16.** Zusammensetzung nach Anspruch 14, wobei n und R in jedem der Konjugate gleich sind.

**17.** Zusammensetzung nach Anspruch 14, wobei n von 450 bis 490 ist.

**18.** Zusammensetzung nach Anspruch 14, wobei in jedem der Konjugate der Granulocyten-Kolonie-stimulierende Faktor GCSF-Mutein ist, das die in Figur 1 gezeigte Sequenz hat.

**19.** Zusammensetzung nach Anspruch 14, wobei R Methyl ist; n in jedem der Konjugate gleich ist und eine ganze Zahl von 450 bis 490 ist; G GCSF-Mutein ist, das die in Figur 1 gezeigte Sequenz hat, abzüglich der Aminogruppen desselben, die eine Amidbindung mit einer Polyethylenglycol-Einheit im Konjugat bilden; in jedem der Konjugate m unabhängig voneinander eine ganze Zahl von 1 bis 4 ist; der prozentuale Anteil der Konjugate, bei denen m 1 ist, von 18 bis 25% beträgt; der prozentuale Anteil an Konjugaten, bei denen m 2 ist, von 50 bis 66% beträgt; der prozentuale Anteil an Konjugaten, bei denen m 3 ist, von 12 bis 16% beträgt; und der prozentuale Anteil an Konjugaten, bei denen m 4 ist, bis zu 5% beträgt.

**20.** Verfahren zum Herstellen eins PEG-GCSF-Konjugats, das eine längere Zirkulations-Halbwertszeit hat und eine größere *in vivo*-granulopoietische Aktivität, als das entsprechende, nicht-konjugierte GCSF, wobei das Verfahren aus einem kovalenten Verbinden eines Reagenzes der Formel

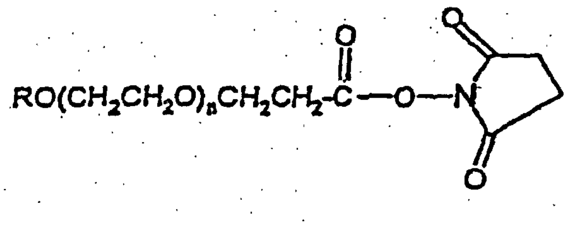

II

an das GCSF besteht, um das Konjugat herzustellen.

**21.** Verfahren nach Anspruch 20, wobei das GCSF GCSF-Mutein ist, das die in Figur 1 gezeigte Sequenz hat.

**Revendications**

**1.** Conjugué physiologiquement actif ayant la formule :

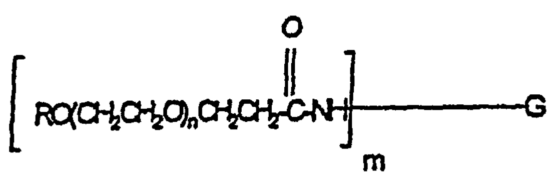

I

dans laquelle G est un facteur de stimulation de colonies de granulocytes moins le groupe ou les groupes amino de celui-ci qui forment une liaison amide avec une fraction de polyéthylèneglycol dans le conjugué ; R est un alkyle inférieur ; n est un nombre entier de 420 à 550 ; et m est un nombre entier de 1 à 5.

**2.** Conjugué de la revendication 1, dans lequel R est du méthyle.

**3.** Conjugué de la revendication 2, dans lequel n est de 450 à 490.

**4.** Conjugué de la revendication 2, dans lequel m est un nombre entier de 1 à 4.

**5.** Conjugué de la revendication 4, dans lequel m est 2.

**6.** Conjugué de la revendication 1, dans lequel le facteur de stimulation de colonies de granulocytes est GCSF mutéine ayant la séquence montrée sur la figure 1.

**7.** Conjugué de la revendication 1, dans lequel n est de 450 à 490.

**8.** Conjugué de la revendication 1, dans lequel m est un nombre entier de 1 à 4.

**9.** Conjugué de la revendication 8, dans lequel m est 2.

**10.** Conjugué de la revendication 1, dans lequel le facteur de stimulation de colonies de granulocytes est GCSF mutéine ayant la séquence montrée sur la figure 1.

**11.** Conjugué de la revendication 1, qui présente une demi-vie de circulation plus longue et une activité granulopoïétique in vivo plus grande que le facteur de stimulation de colonies de granulocytes non conjugué correspondant.

**12.** Conjugué de la revendication 11, dans lequel le facteur de stimulation de colonies de granulocytes est GCSF mutéine ayant la séquence montrée sur la figure 1.

**13.** Conjugué selon la revendication 1, dans lequel R est du méthyle ; n est un nombre entier de 450 à 490 ; m est 2 ; et G est GCSF mutéine ayant la séquence montrée sur la figure 1 moins les groupes amino de celui-ci qui forment une liaison amide avec une fraction de polyéthylèneglycol dans le conjugué.

**14.** Composition comprenant des conjugués physiologiquement actifs ayant la formule :

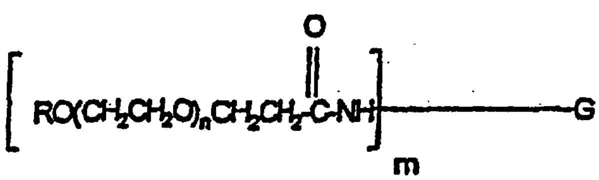 I

dans laquelle G dans chacun des conjugués est un facteur de stimulation de colonies de granulocytes moins le groupe amino ou les groupes amino de celui-ci qui forment une liaison amide avec une fraction de polyéthylèneglycol dans les conjugués ; R dans chacun des conjugués est indépendamment un alkyle inférieur ; n dans chacun des conjugués est indépendamment un nombre entier de 420 à 550 ; dans chacun des conjugués m est indépendamment un nombre entier de 1 à 4 ; le pourcentage des conjugués où m est 1 est de 18 à 25% ; le pourcentage des conjugués où m est 2 est de 50 à 66% ; le pourcentage des conjugués où m est 3 est de 12 à 16% ; et le pourcentage des conjugués où m est 4 est jusqu'à 5%.

**15.** Composition de la revendication 14, dans laquelle R est du méthyle dans chacun des conjugués.

**16.** Composition de la revendication 14, dans laquelle n et R sont les mêmes dans chacun des conjugués.

**17.** Composition de la revendication 14, dans laquelle n est de 450 à 490.

**18.** Composition de la revendication 14, où dans chacun des conjugués le facteur de stimulation de colonies de granulocytes est GCSF mutéine ayant la séquence montrée sur la figure 1.

**19.** Composition selon la revendication 14, dans laquelle R est du méthyle ; n dans chacun des conjugués est le même et est un nombre entier de 450 à 490 ; G est GCSF mutéine ayant la séquence montrée sur la figure 1 moins les groupes amino de celui-ci qui forment une liaison amide avec une fraction de polyéthylèneglycol dans le conjugué ; dans chacun des conjugués m est indépendamment un nombre entier de 1 à 4 ; le pourcentage des conjugués où m est 1 est de 18 à 25% ; le pourcentage des conjugués où m est 2 est de 50 à 66% ; le pourcentage des

conjugués où m est 3 est de 12 à 16% ; et le pourcentage des conjugués où m est 4 est jusqu'à 5%.

**20.** Procédé pour produire un conjugué PEG-GCSF ayant une demi-vie de circulation plus longue et une activité granulopoïétique in vivo plus grande que le GCSF non conjugué correspondant, lequel procédé consiste à lier de façon covalente un réactif de formule

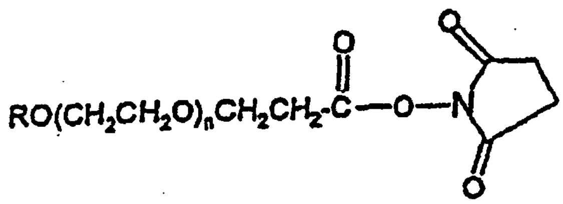

II

au GCSF pour produire ledit conjugué.

**21.** Procédé selon la revendication 20, dans lequel le GCSF est GCSF mutéine ayant la séquence montrée sur la figure 1.

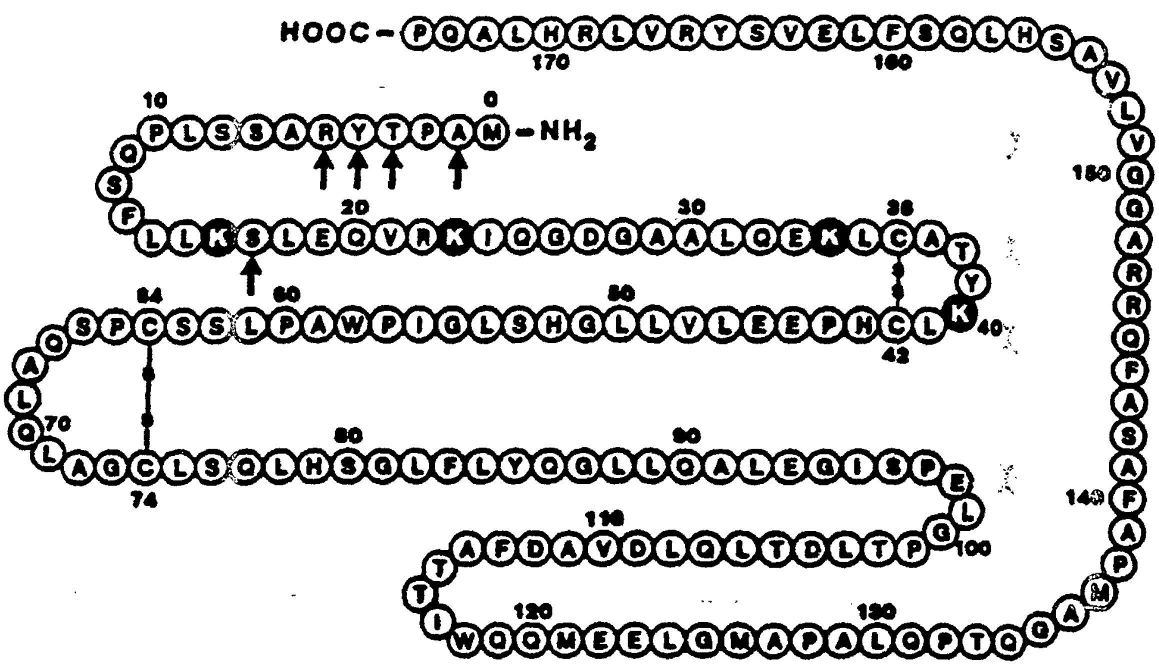


| rhG-CSF | | G-CSF MUTEIN |
|---|---|---|
| $Thr^1$ | → | $Ala^1$ |
| $Leu^3$ | → | $Thr^3$ |
| $Gly^4$ | → | $Tyr^4$ |
| $Pro^5$ | → | $Arg^5$ |
| $Cys^{17}$ | → | $Ser^{17}$ |

Figure 1: Primary structure of GCSF MUTEIN

# Figure 2. Pegylation Reagents

A. GABA, Amide

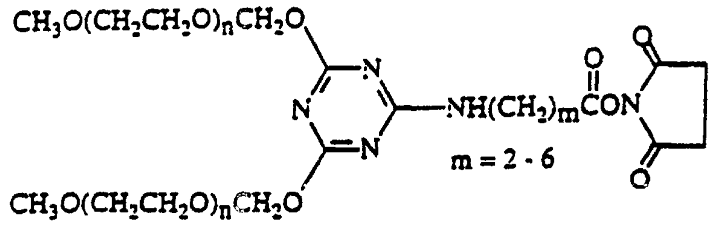

B. Amide

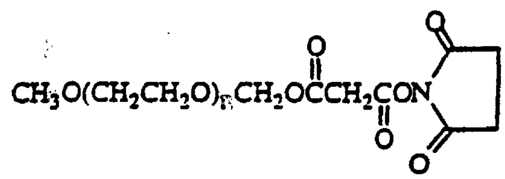

C. Amide (SPA)

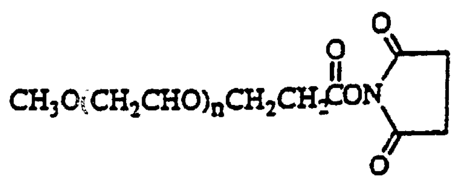

D. Urea

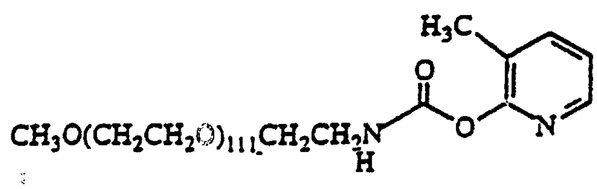

E. Urethane

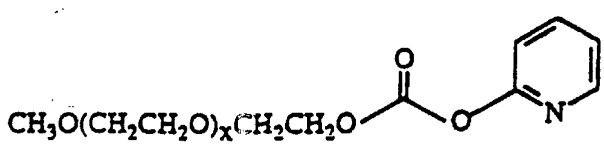

F. Thio-Urethane

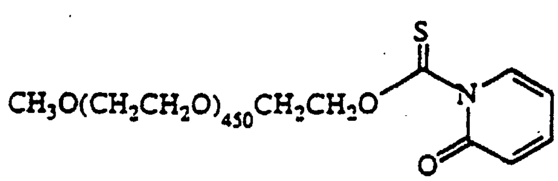

G. Urethane

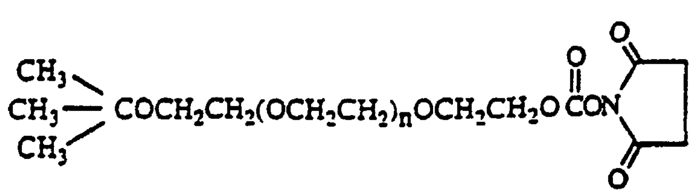

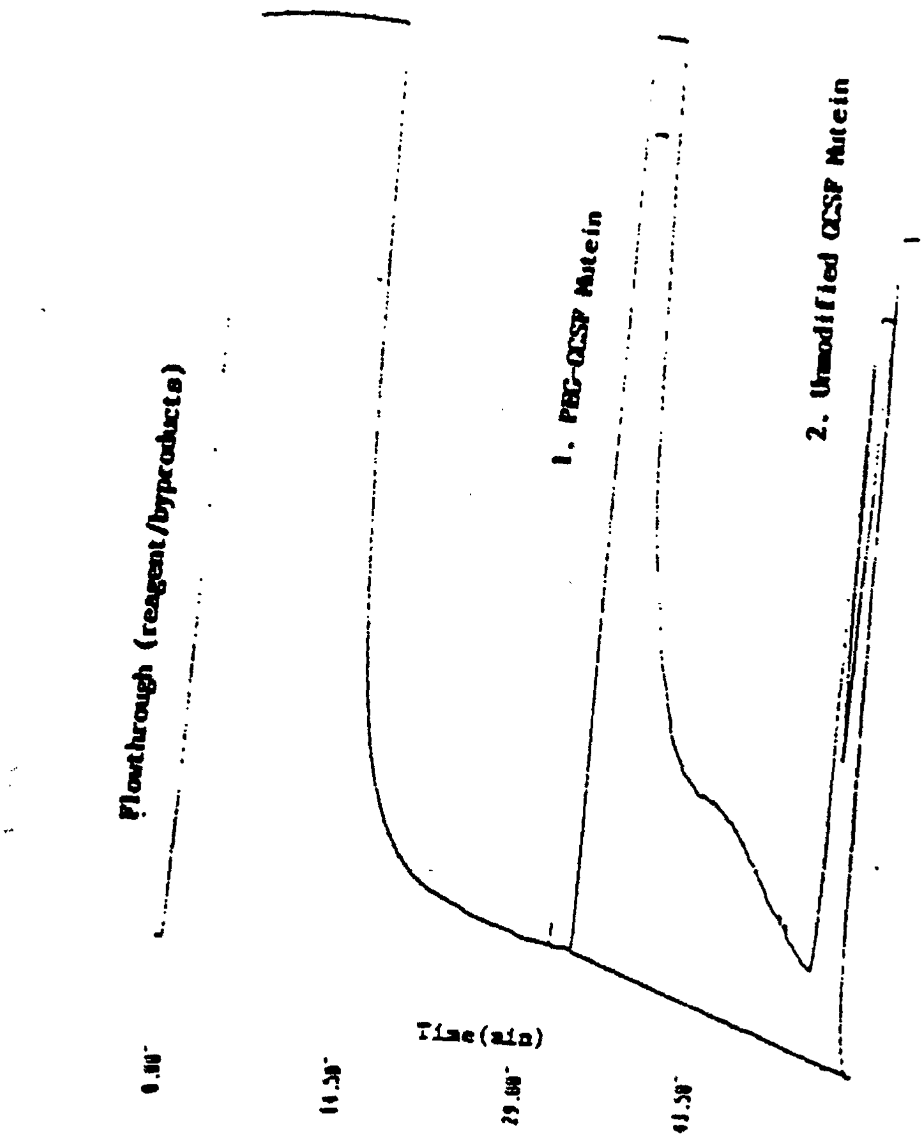


Figure 3 Separation of 20kDa PEG-modified and unmodified GCSF Mutein.
A typical elution profile for PEG reaction mixture.
Column: 1-2ml Fractogel® EMD $SO_3$ 650S.

| | |
|---|---|
| Equilibration Buffer: | 10mM Ammonium Acetate. pH 4.5 |
| Elution Buffers: 1. | 0.15M NaCl in equilibration buffer |
| 2. | 0.5M NaCl in equilibration buffer |

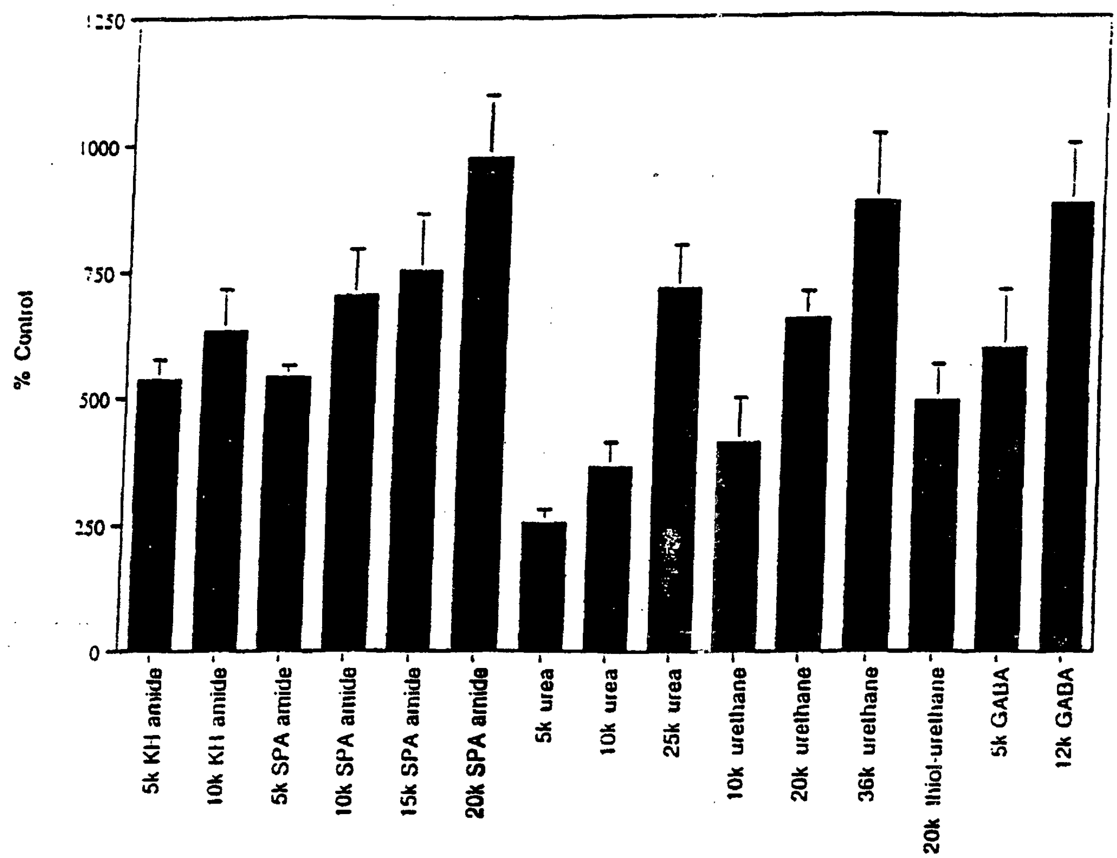


Figure 4: PEG-GCSF Mutein Activity on Day 5 after a Single Injection

Female C57BL/6J mice were injected subcutaneously with 25.2μg of the peyglated GCSF Mutein conjugates; on the fifth day following administration, retroobital venous GCSF Muteins samples were collected. Coulter hematological and leukocyte differential analyses were performed; the resulting neutrophil counts were standarized to vehicle control for each experiment. Data represents the mean ± S.E. of 4 mice per group.

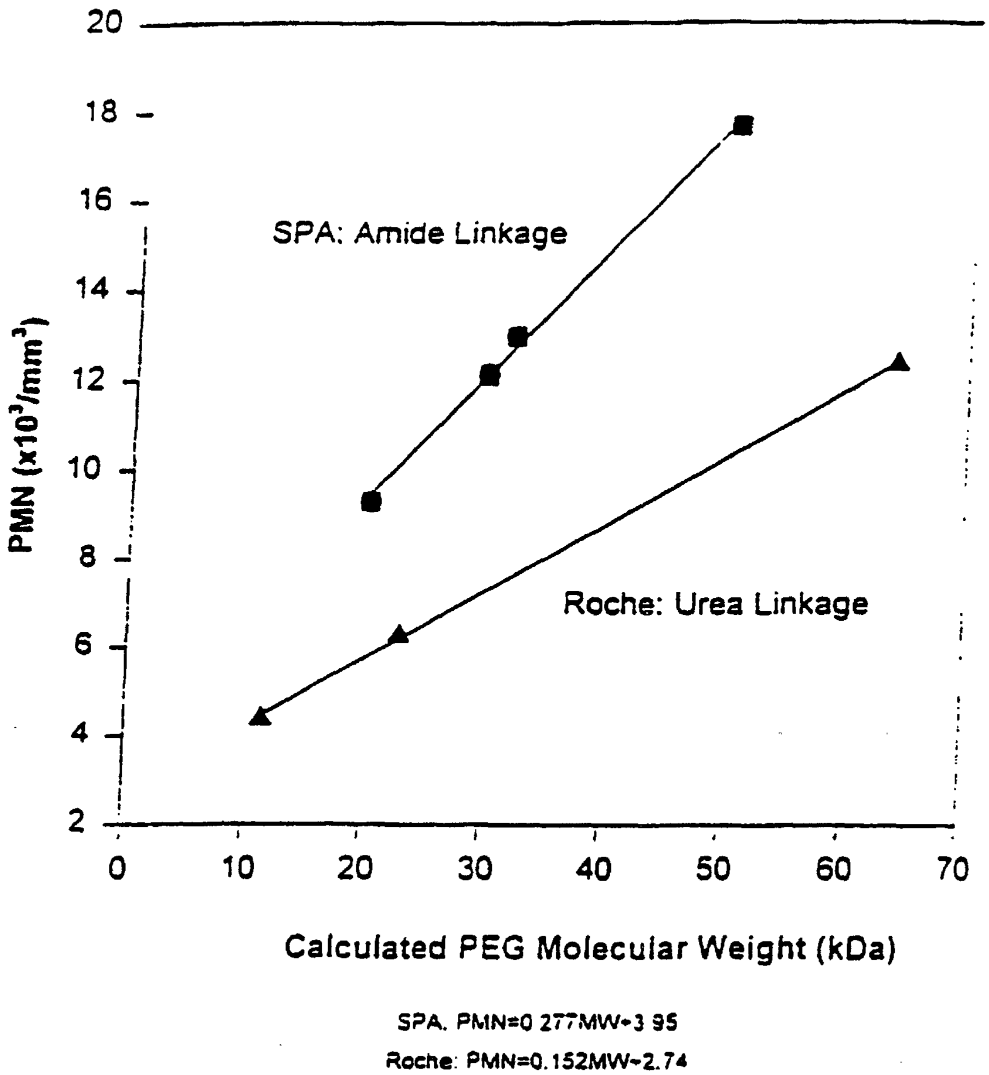


Figure 5. Increase in PMN count as a function of PEG mass (kDa) in amide and urea linked PEG-GSCF Mutein conjugates.

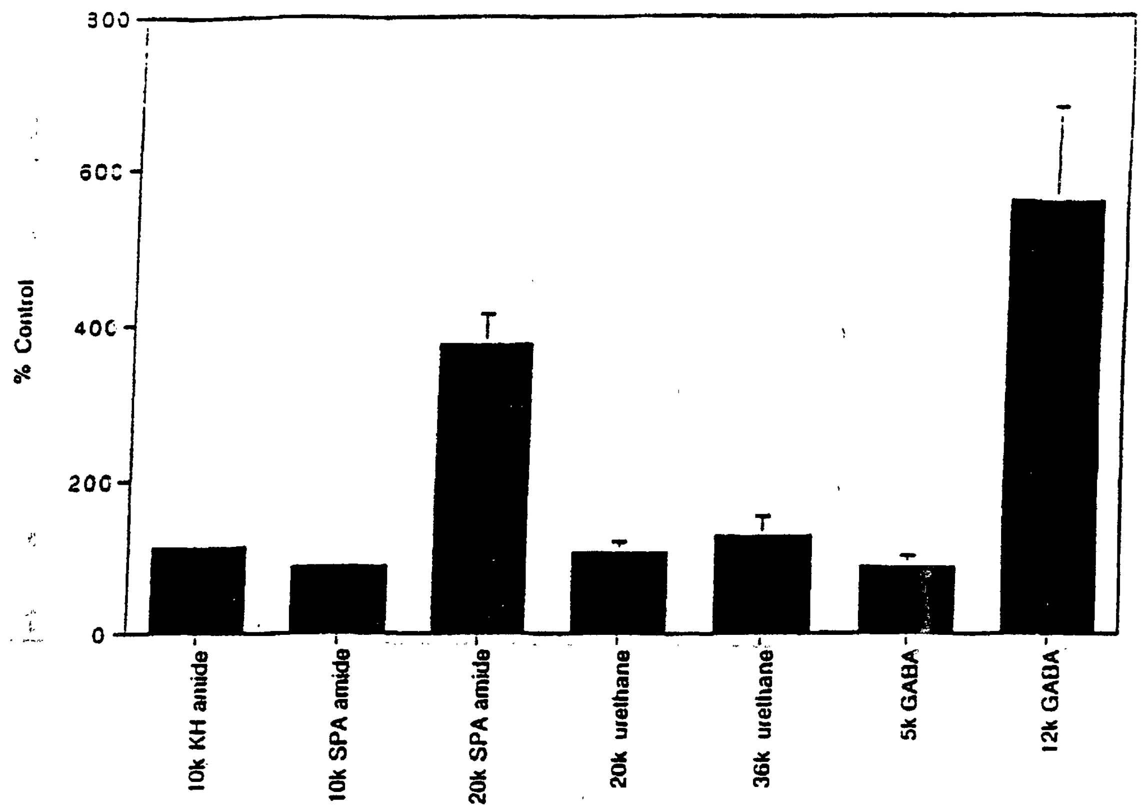


Figure 6: PEG-GCSF Mutein Activity on Day 7 after a Single Injection
Female C57BL/6J mice were injected subcutaneously with 25.2μg of the pegylated conjugates; one the seventh day following administration, retroorbital venous blood samples were collected. Coulter hematological and leukocyte differential analyses were performed; the resulting neutrophil counts were standarized to vehicle control for each experiment. Data represents the mean ± S.E. of 4 mice per group.

Figure 7

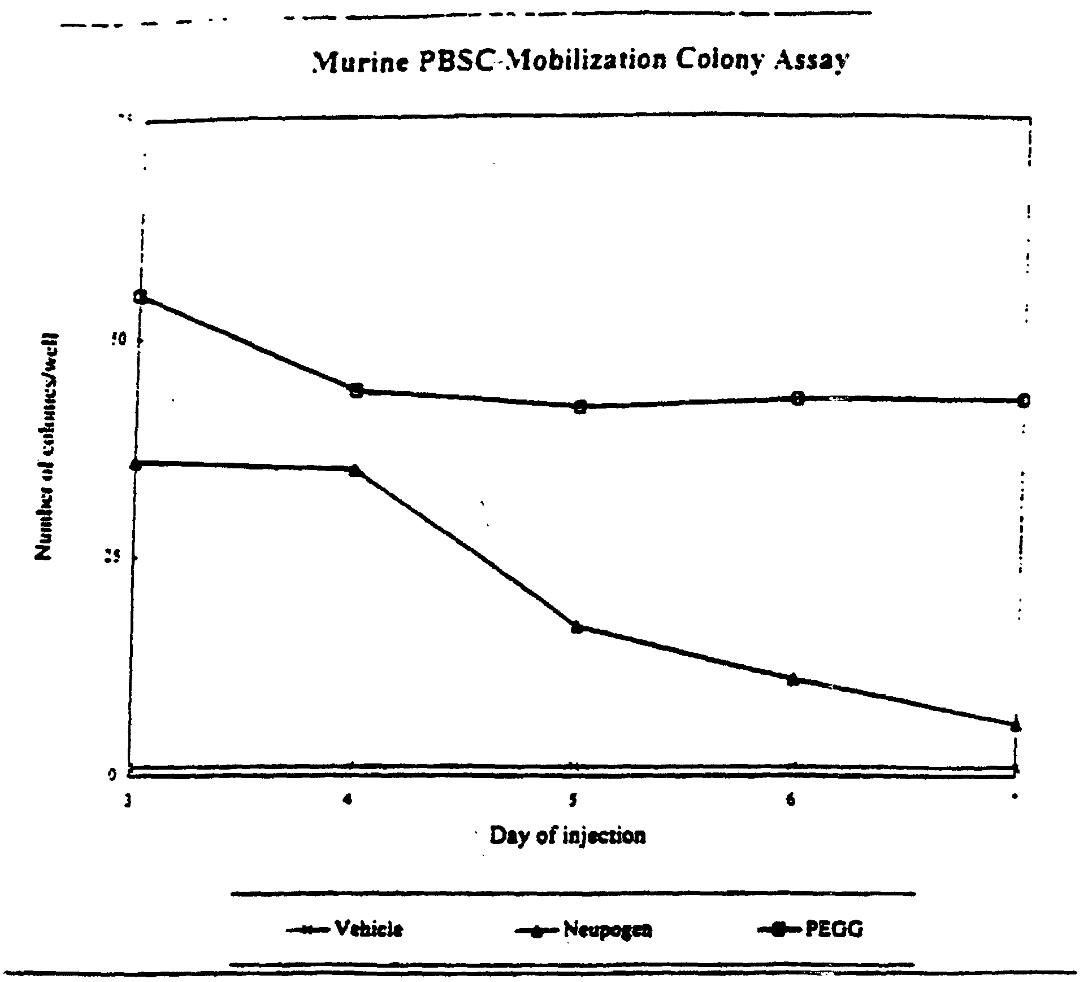


Neupogen (25 µg/mouse) injected on days 0 - 5
PEGG (25 µg/mouse) injected on day 0

Figure 8

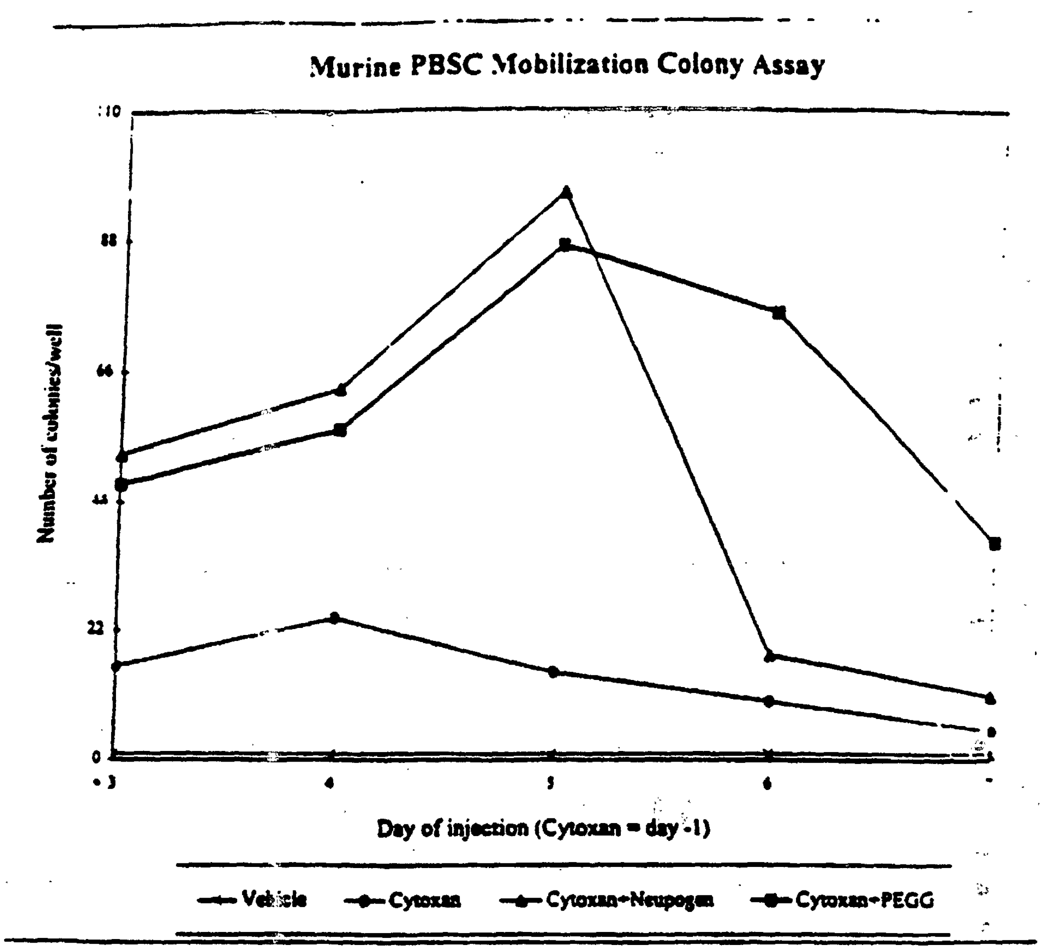


Neupogen (25 μg/mouse) injected on days 0 - 5
PEGG (25 μg/mouse) injected on day 0

Figure 9

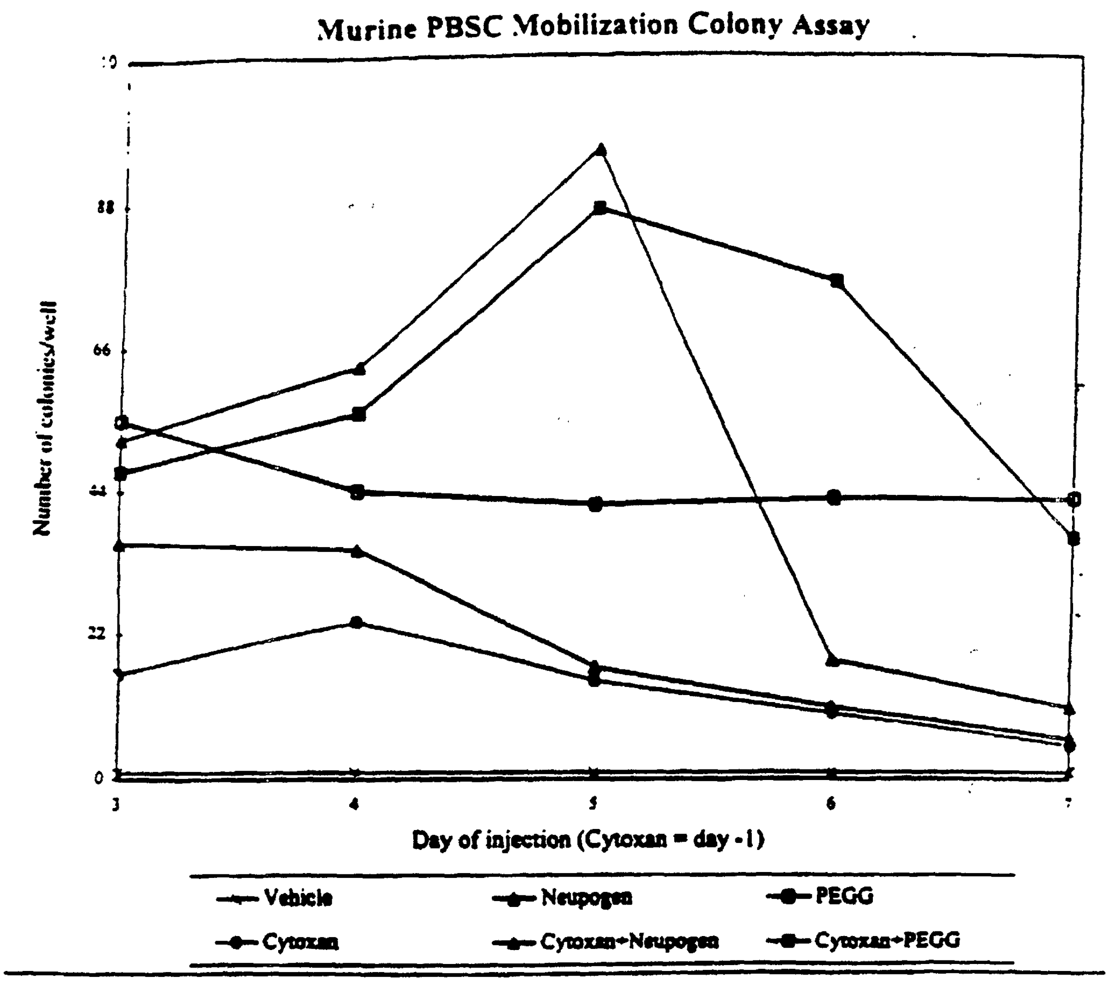


Neupogen (25 µg/mouse) injected on days 0 - 5

PEGG (25 µg/mouse) injected on day 0

Figure 10

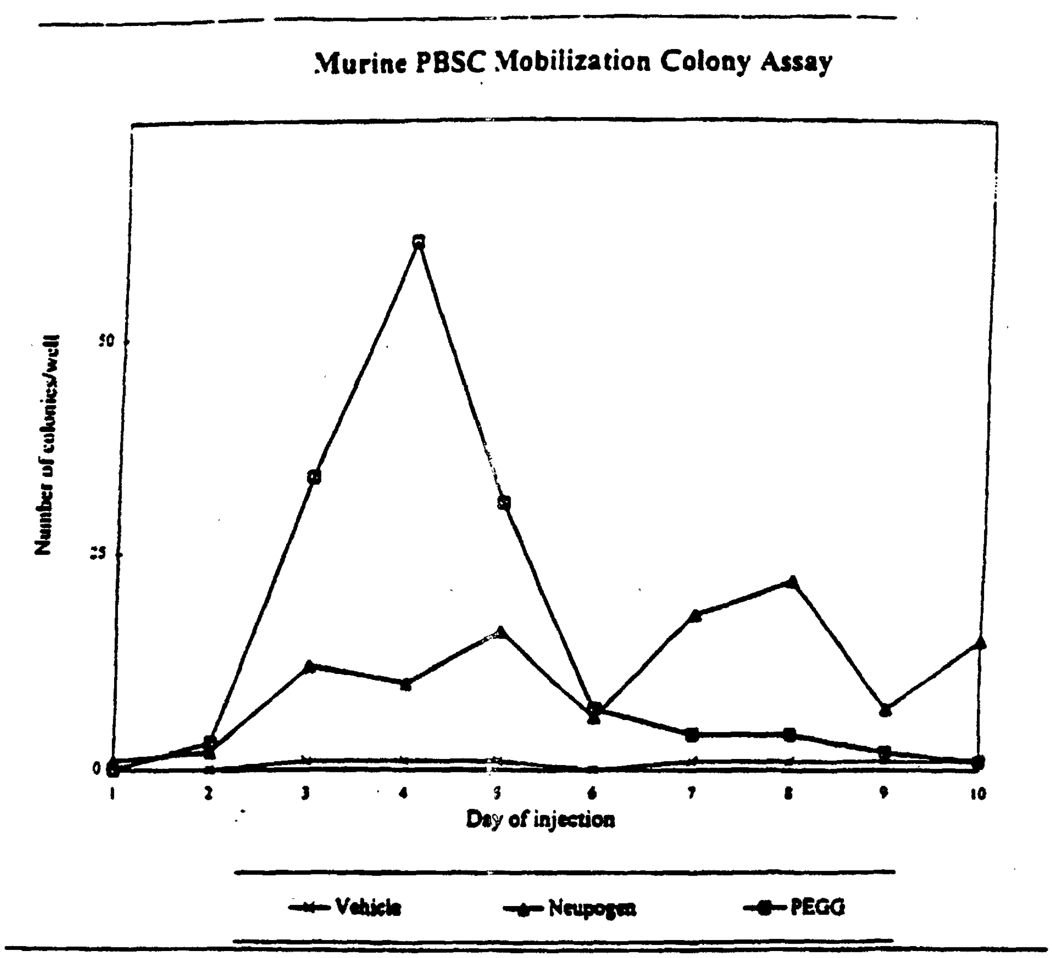


Neupogen (3 µg/mouse) injected on days 0 - 9
PEGG (3 µg/mouse) injected on day 0

Figure 11

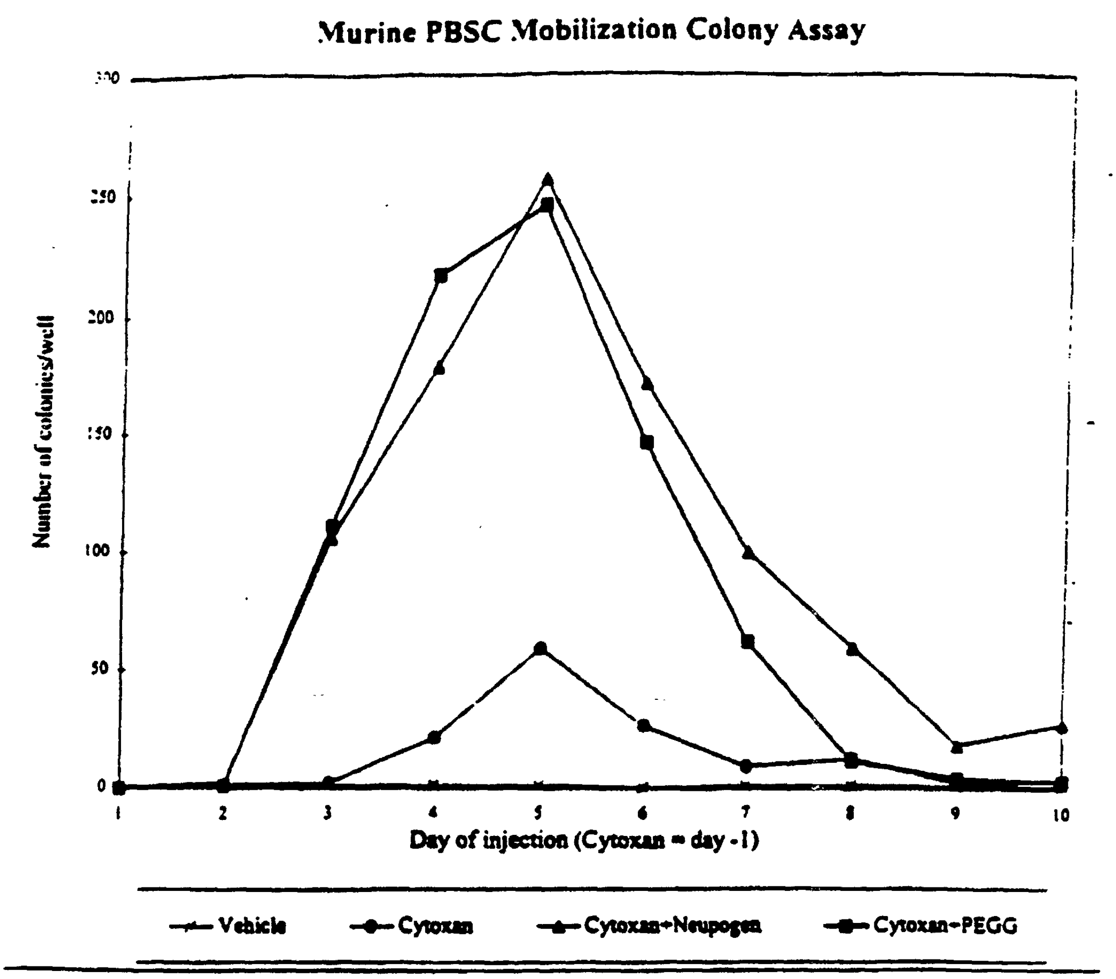


Neupogen (3 μg/mouse) injected on days 0 - 9

PEGG (3 μg/mouse) injected on day 0